# EUROPEAN PATENT APPLICATION

(11) **EP 1 033 121 A2**
(43) Date of publication of application: **06.09.2000**
(21) Application number: 00850021.7
(22) Date of filing: 04.02.2000
(51) Int. Cl.: A61F 13/15

(54) **A method of producing absorbent articles in a continuous production line**

(30) Priority: 01.03.1999 SE 9900735
(71) Applicant: SCA Hygiene Products AB, 405 03 Göteborg (SE)
(72) Inventor: Böhm, Thomas, 426 69 Västra Frölunda (SE); Helmfridsson, Bror Inge, 433 50 Partille (SE); Berthu, Thomas, 416 61 Göteborg (SE); Silverstrand, Anders, 435 44 Mölnlycke (SE)
(74) Representative: Hyltner, Jan-Olof

(57) **Abstract**

The present invention relates to a method of manufacturing in a continuous production line absorbent articles (15) which each comprise a top sheet (1'), a backing sheet (7'), an absorbent body (3) enclosed between the top sheet and the backing sheet, and an adhesive coating (10') provided with a protective layer (11*'*) and fastened to the backing sheet, said method comprising the steps of placing discrete absorbent bodies (3) in a row on a travelling first web (1) of top sheet or backing sheet material, and thereafter placing a travelling second web (7) of backing sheet or top sheet material onto said first web, fastening the first and second webs together at parts which lie outwardly of the absorbent bodies, and thereafter cutting individual absorbent articles from the composite web (1, 3, 7). According to the invention a protective layer (11) that includes an adhesive coating (10) is applied to the web of backing sheet material (7) whilst said web is in a flat state and prior to combining the web with said absorbent bodies (3) and/or the web of top sheet material (1).

## Description

### FIELD OF INVENTION

The present invention relates to a method of manufacturing absorbent articles in a continuous production line, each of said articles comprising a top sheet, a backing sheet, an absorbent body enclosed between said top and backing sheets, and an adhesive coating provided with a protective layer and fastened to the backing sheet, wherein the method comprises the steps of laying a line of mutually separate absorbent bodies on a travelling first web of top-sheet or backing-sheet material, laying a travelling second web of backing-sheet or top-sheet material on top of the first web, joining the first and the second webs together at parts thereof which lie outwardly of the absorbent bodies, and then separating individual absorbent articles from the resultant composite web.

### BACKGROUND OF THE INVENTION

Certain types of absorbent articles, such as sanitary napkins and incontinence protectors, often include adhesive coatings for fastening the article to the panties or underpants of the user. One problem with articles of this kind is that adhesive residues can remain in the wearer's panties or underpants after removing the article.

The object of the present invention is to solve this problem.

### SUMMARY OF THE INVENTION

This object is achieved with a method of producing absorbent articles in a continuous production line where each article includes a top sheet, a backing sheet, an absorbent body enclosed between said top sheet and said backing sheet, and an adhesive coating provided with a protective layer and fastened to the backing sheet, wherein the method comprises the steps of laying a line of mutually separated absorbent bodies on a travelling first web of top-sheet or back-sheet material, thereafter laying a travelling second web of backing-sheet material or top-sheet material on top of the first web and fastening the first and the second webs together at parts which lie outwardly of the absorbent bodies, and thereafter separating the absorbent articles from the composite web, characterised by applying an adhesive coating that includes a protective layer to the web of backing-sheet material whilst it is in a flat state and prior to it being combined with the absorbent bodies and/or the web of top-sheet material.

In one preferred embodiment, the adhesive coating is applied continuously and the web of backing-sheet material is rolled-up on a storage reel and the adhesive coating is applied to the web of backing-sheet material subsequent to unreeling said material from its storage reel and prior to introducing said material into the production line.

In one variant, the adhesive coating is applied intermittently.

### BRIEF DESCRIPTION OF THE DRAWING

The present invention will now be described with reference to the accompanying drawing, in which
Fig. 1 illustrates schematically an absorbent article production line according to one embodiment of the invention; and
Fig. 2 is a view from above of an absorbent article manufactured in the production line illustrated in Figure 1, with that side of the article which lies proximal to the panties or underpants of a user facing towards the viewer.

### DESCRIPTION OF EMBODIMENTS

The inventors have based their invention on the realisation that adhesive residues will remain in a pair of panties or underpants after removing a sanitary napkin or an incontinence protector therefrom because of the presence of local air bubbles between the backing sheet and the adhesive coating, which prevent the adhesive coating from fastening over the whole of the surface of the backing sheet of said article. In turn, this is because in a conventional production line the backing sheet is adapted to the surface of the absorbent body, this surface often being irregular due to the presence of embossments, impressions, grooves and the like formed in the absorbent body. In order for the adhesive coating to accompany the article when it is removed from the wearer's panties, it is necessary for the glues or adhesives used in sanitary napkins and like articles to have properties such tat the glue will have stronger adhesion to the backing sheet than to the material from which the panties or underpants are made. Air bubbles that are enclosed between the glue layer and the backing sheet prevent the glue from being pressed against the backing sheet in the regions of the air bubbles. In the regions of the air bubbles, the glue adheres solely to the panties or underpants of the wearer of the article, therewith making it highly probably that glue residues will remain on the panties or underpants of the wearer when the article is removed.

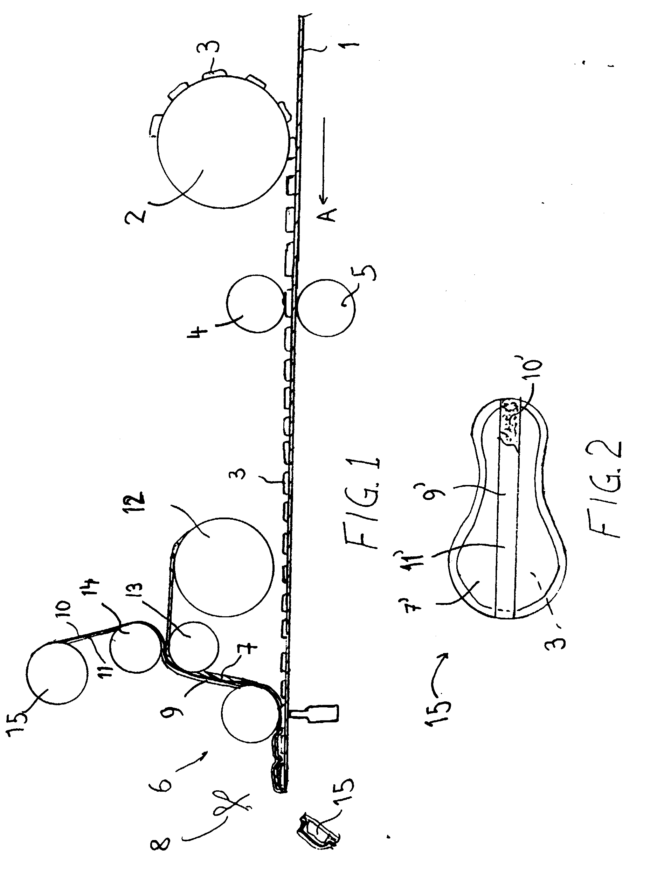

Figure 1 is a schematic illustration of an arrangement for the continuous manufacture of said sanitary napkins which ensures that air bubbles will not be enclosed between an adhesive coating and the backing sheet.

The arrangement illustrated in Figure 1 includes in a well known manner one or more transporters, e.g. vacuum transporters, (not shown) for feeding a web 1 of top sheet material through the production line in the direction indicated by the arrow A in Figure 1. The web 1 is taken from a storage reel (not shown) and passes a first device 2 which functions to apply a line or row of mutually separate absorbent bodies 3 on the web 1. The device 2 may comprise an absorbent body transfer wheel which collects absorbent bodies from a mat-forming wheel (not shown) and delivers said bodies 3 to the web 1. The mat-forming wheel and transfer wheel are well known to the person skilled in this art and need not be described in detail here. After passing the device 2, the composite web 1, 3 comprising absorbent bodies and top sheet material passes through a pair of rolls 4, 5. The roll 4 has a patterned surface and when exiting from the nip between the rolls 4, 5 the absorbent bodies 3 will have an embossed surface on that side which faces away from the top sheet material 1. The composite web 1, 3 then passes through a device which applies a web 7 of backing material to the web 1, 3 and fastens said web 7 to the web 1 at parts thereof which lie outside the absorbent bodies 3. The device 6 may be an ultrasound welding unit, as indicated schematically in Figure 1. The composite web comprised of said webs 1, 7 and the absorbent bodies enclosed therebetween then passes a cutting or punching tool 8 which separates individual absorbent articles 15 from the composite web.

Prior to the web 7 of backing material entering the ultrasound welding unit 6, a continuous strip 9 of release paper 11 that includes a hotmelt glue coating 10 is fastened to the web 7 of backing material. In the case of the embodiment illustrated in Figure 1, the web 7 is taken from a storage reel 12 and passed through the nip between two rolls 13, 14. The strip 9 is rolled-up onto a storage reel 15. The strip 9 also passes from the reel 15 through the nip defined by the rolls 13, 14. In order the ensure that the hotmelt glue will fasten properly to the web 7 of backing material, the roll 14 is suitably heated so that the glue will be sticky when applied to the web 7. In order to prevent the occurrence of wrinkles or folds in the web 7 during its passage through the rolls 13, 14, a web tensioning unit may be arranged in the proximity of the rolls 13, 14, particularly when the storage reel 12 is located at a fair distance from the rolls 13, 14. This ensures that the web will always be smooth when applying the hotmelt glue coating in the nip between the rolls 13, 14.

Figure 2 illustrates schematically and from above a sanitary napkin 15 manufactured by means of the arrangement shown in Figure 1. The viewer sees the napkin from that side which is fastened to the wearer's panties in use. The reference signs used to identify the components of the napkin 15 are the same as those used in Figure 1 but with the addition of a prime. Thus, the sanitary napkin 15 shown in Figure 2 includes a top sheet 1', which is not visible in the Figure 2 illustration, a backing sheet 7' and an absorbent body 3 enclosed therebetween. The top sheet and the backing sheet are joined together at parts which lie outwardly of the absorbent body. A strip 10' of hotmelt glue extends longitudinally over the backing sheet 7' and is covered with a release layer 11'.

The top sheet 1 is comprised of liquid-permeable material, for instance a non-woven material or perforated plastic film, and the backing sheet is comprised of liquid-impermeable material, e.g. plastic film. The absorbent body is comprised of one or more layers of cellulose fluff, either with or without an admixture of particles of so-called superabsorbent material. The hotmelt glue may consist of Ecomelt H 145 from Collano AG, Switzerland, although other glues may alternatively be used, for instance Lunatack D58 ZP from HB Fuller Gmbh, Germany. The release paper is comprised of a conventional silicone-coated paper.

Because the non-stretchable release paper is continuous in the case of the illustrated embodiment, the risk of wrinkles or folds forming during the manufacturing process is reduced, since the release paper prevents stretching of the web of backing material.

In one variant of the method, a line or row of discrete glue-coated strips are applied to the web of backing material prior to said material being introduced into the process line. This can be achieved, for instance, by transferring the continuous web of glue-coated release paper in Figure 1 from the storage reel 15 to a first vacuum conveyor which includes a row of slides or like devices which are placed closely adjacent one another at a first end of the conveyor and which separate from each other as they move from said first end to the second end of the conveyor and therewith separate said web into individual strips in the process. When the row of individual strips reaches the second end of the first conveyor, they are transferred to a second conveyor which passes through the nip defined by the rolls 13, 14.

The described embodiment may, of course, be modified within the scope of the invention. For instance, more than two continuous webs of material may be included in the process line. For instance, the web of backing material may consist of a plastic film/non-woven laminate, wherewith the glue-coated release paper strip is fastened to the non-woven material either before being fastened to the plastic film layer or subsequent thereto. Furthermore, the absorbent articles may have shapes other than the hourglass shape shown in Figure 2, and the top sheet and backing sheet can form outwardly projecting flaps or wings at the centre part of the article. Release material other than silicone-coated paper may be used as a protective layer for the glue coating and adhesives other than hotmelt glue may be used. The manufacturing process may also include more steps than those described. For instance, the manufacturing line may include means for fastening pre-stretched elastic threads. It is also possible, of course, to place absorbent bodies and top sheets on the web of backing sheet material instead of supplying the web of backing material in the final stage of the manufacturing process, in which case the web of backing sheet material will have been provided with a continuous strip or a row of strips of glue-coated release paper before the row of absorbent bodies is placed on the web. As will be understood, it is also possible to coat a web of release paper with adhesive in conjunction with applying the adhesive-coated web to the backing sheet. It is also possible to apply the web of release paper and adhesive beforehand and to roll the release paper onto a storage reel which can later be moved to the manufacturing line. The invention is therefore restricted solely by the contents of the following Claims.

## Claims

1. A method of manufacturing in a continuous production line absorbent articles (15) which each comprise a top sheet (1'), a backing sheet (7'), an absorbent body (3) enclosed between the top sheet and the backing sheet, and an adhesive coating (10') provided with a protective layer (11*'*) and fastened to the backing sheet, said method comprising the steps of placing discrete absorbent bodies (3) in a row on a travelling first web (1) of top sheet or backing sheet material, and thereafter placing a travelling second web (7) of backing sheet or top sheet material onto said first web, fastening the first and second webs together at parts which lie outwardly of the absorbent bodies, and thereafter cutting individual absorbent articles from the composite web (1, 3, 7), **characterised** by applying a protective layer (11) that includes an adhesive coating (10) onto the web of backing sheet material (7) whilst said web is in a flat state and prior to combining the web with said absorbent bodies (3) and/or the web of top sheet material (1).

2. A method according to Claim 1, **characterised** by applying the adhesive coating (10) continuously.

3. A method according to Claim 1, **characterised** by applying the adhesive coating intermittently.

4. A method according to any one of Claims 1-3, **characterised** by rolling the web (7) of backing sheet material onto a storage reel (15) and applying the adhesive coating (10) to said web of backing sheet material subsequent to having reeled said web from said storage reel and prior to introducing said web into the production line.
